# EUROPÄISCHE PATENTANMELDUNG

(11) **EP 1 419 787 A1**
(43) Veröffentlichungstag der Anmeldung: **19.05.2004**
(21) Anmeldenummer: 02025600.4
(22) Anmeldetag: 18.11.2002
(51) Int. Cl.: A61K 49/00

(54) **Lymphknoten-Kontrastmittel**

(71) Anmelder: Till, Uwe Prof. Dr., 99087 Erfurt (DE)
(72) Erfinder: Till, Uwe Prof. Dr., 99087 Erfurt (DE)
(74) Vertreter: HOFFMANN - EITLE

(57) **Zusammenfassung**

Die Erfindung betrifft eine Zusammensetzung enthaltend pharmazeutisch akzeptable Partikel, die zu 90 % oder mehr eine Teilchengröße im Bereich von 5 bis 1500 nm aufweisen und mit einem Farbstoff angefärbt sind, der sich von der Farbe menschlichen Gewebes leicht unterscheiden lässt. Die Zusammensetzung wird als Kontrastmittel für die Lymphknoten-Darstellung, insbesondere von Sentinel-Lymphknoten, verwendet.

## Beschreibung

Die Erfindung betrifft ein Kontrastmittel auf Farbstoffbasis für die Lymphknoten-Darstellung, und zwar insbesondere die Darstellung von Sentinel-Lymphknoten. Solche Kontrastmittel dienen beispielsweise einem Chirurgen während einer Tumoroperation als wichtiges Hilfsmittel für die Erkennung und anschließende Entfernung eines Lymphknotens. Ferner betrifft die Erfindung Verfahren zur Herstellung solcher Kontrastmittel und deren Verwendung als Diagnostika in der Medizin.

### Hintergrund der Erfindung

Die häufigsten bösartigen Tumoren des Menschen setzen ihre Tochtergeschwülste primär in dem zugehörigen Lymphabflussgebiet ab. Auf diese Weise entstehen Lymphknotenmetastasen. Bei der operativen Tumorentfernung werden daher in der Regel die Tumor-benachbarten Lymphknotenstationen so komplett wie möglich entfernt. Nachfolgend werden die entfernten Lymphknoten in der Pathologie auf Metastasen untersucht. Von dem Ergebnis hängt die weitere Behandlung ab. Neben dem operativen Aufwand und der großen Zahl histomorphologischer Untersuchungen kann dieses Verfahren vor allem nach der Operation mit einer erheblichen gesundheitlichen Beeinträchtigung der Patienten verbunden sein, wie etwa durch Behinderung des Lymphabstroms, Narbenbildung, Nervenschädigung usw.

Nach dem sogenannten Sentinel(=Wächter)-Lymphknoten-Konzept wird bei der Bildung von Tochtergeschwülsten primär immer der Lymphknoten befallen, der als erster im Abstrombereich des Tumors stationiert ist (Büchels HK, Vogt H, Wagner T, Steinfeld D, Sagassser J, Sentinel-Lymphonodektomie beim Mammakarzinom, Der Nuklearmediziner 1999, 22; 261-267). Bei Beschränkung auf die Entfernung dieses Knotens (oder dieser Knoten bei mehreren Abstromgebieten) und den während der Operation durch sogenannten Schnellschnitt geführten Nachweis einer Metastasenfreiheit dieses Knotens könnte die Operation in diesem Stadium beendet werden. Der operative und histomorphologische Aufwand und die Beeinträchtigung der Patienten wären somit weitaus geringer. Dieses "Sentinel-Lymphknotenprinzip" ist derzeit für die operative Entfernung von Melanomen weitgehend etabliert und befindet sich für viele andere Turmorarten in klinischer Erprobung (Vogt H, Wengenmair H, Kopp J, Dorn R, Gröber S, Heidenreich P, Der Sentinel-Lymphknoten (SLN): prä- und intraoperative nuklearmedizinische Diagnostik, Der Nuklearmediziner 1999, 22, 233-242; Balch CM, Lange JR, Lymphatic Mapping and Sentinel Node Lymphadenectomy for Cancer: An Overview, Annals of Surgical Oncology 2001, 8(9S), 1-4; Zervos EE, Burak WE, Lymphatic Mapping in Solid Neoplasms: State of the Art, Cancer Control 2002, (8)3, 189-202).

Zur Erfassung von Sentinel-Lymphknoten werden derzeit vor allem zwei methodische Prinzipien eingesetzt:

Zum einen werden in Lösung befindliche kolloidale Partikel durch Technetium-99m radioaktiv markiert und vor der Operation in die unmittelbare Umgebung des Tumors injiziert. Die Partikel werden dann über die Lymphwege abtransportiert und in dem bzw. den Sentinel-Lymphknoten gespeichert. Ihr Nachweis erfolgt meist in zwei Schritten: a) am Vortag der Operation durch Nachweis der Radioaktivität mittels Ganzkörperszintigrafie und b) während der Operation mit eigens dafür konstruierten Gamma-Sonden als Radioaktivitätsdetektoren. Die Nachteile dieser Methode sind allerdings beträchtlich (siehe Heidenreich P, Vogt H, Bachter D, Büchels H, Steinfeld D, Wawroschek F, Wengenmair H, Wagner T, Das Konzept des Wächterlymphknotens, Dt. Ärzteblatt 2001, 98; A534-540):
- Sie ist an das Vorhandensein einer nuklearmedizinischen Abteilung gebunden.
- Sie ist technisch außerordentlich aufwändig. Neben der Ganzkörperszintigrafie sind speziell konstruierte Gamma-Sonden notwendig, die je nach Operationsgebiet verschieden sein müssen.
- Sie ist mit einer Strahlenexposition von Operationsteam, Patienten und Pathologen verbunden und erfordert entsprechende Vorkehrungen.
- Die eindeutige Lokalisation des Sentinel-Lymphknotens während der Operation ist mitunter nicht möglich, wegen zu enger Verhältnisse oder wenn der Sentinel-Lymphknoten sehr nahe dem Injektionsort liegt, an dem die meiste Radioaktivität liegen bleibt und so den Lymphknoten überstrahlt.

Trotz dieser Nachteile wird - in Ermangelung besserer Alternativen - diese Methode gegenwärtig in vielen Krankenhäusern angewendet.

Zum anderen werden für die Lymphknotendarstellung biokompatible, blaue Farbstoffe in die unmittelbare Umgebung des Tumors kurz vor der Operation injiziert. Dadurch färben sich Lymphbahnen und -knoten. Aber auch diese Methode hat ihre Nachteile (Bachter D, Starz H, Volkmar C, Vogt H, Büchels H, Balda BR, Die Sentinel-Lymphonodektomie beim malignen Melanom, Der Nuklearmediziner 1999, 22; 245-252):
- Die gegenwärtig eingesetzten Farbstoffe färben zwar zunächst die Sentinel-Lymphknoten, werden dort aber nicht komplett zurückgehalten, sondern passieren sie. Sie färben daher später auch andere, entfernter liegende Lymphknoten, so dass der Sentinel-Lymphknoten nicht immer eindeutig festgestellt werden kann.
   Dadurch ist der Operateur unter Umständen gezwungen, unnötig viele Lymphknoten zu entfernen, was mit einer entsprechenden post-operativen Belastung für den Patienten verbunden ist.
- Die Farbstoffe gehen in das Blut über und werden über Galle und Urin ausgeschieden. Es kommt zur Blaufärbung der Patienten, die einige Tage anhalten kann.

Die Nachteile der beiden Verfahrensweisen können teilweise durch die Kombination beider Techniken vermindert werden, indem z.B. eine grobe Orientierung durch die Gamma-Sonde durch die Färbung der Lymphknoten verbessert wird (Zervos EE, Burak WE, Lymphatic Mapping in Solid Neoplasms: State of the Art, Cancer Control 2002, (8)3, 189-202). Mit einer solchen Kombination wurden bislang die besten Ergebnisse erreicht (Allan R, Sentinel node localization: do or dye alone? Br J Radiol 2001, 74; 475-477). Der notwendige apparative und präparative Aufwand ist aber dann sehr hoch.

### Zusammenfassung der Erfindung

Ausgehend von diesem Stand der Technik ist es das der Erfindung zugrundeliegende Ziel, eine Zusammensetzung für ein Verfahren der Lymphknotendarstellung bereitzustellen, das nicht die Nachteile der oben diskutierten, bekannten Verfahren aufweist. Insbesondere soll ein ebenso zuverlässiges und weniger aufwändiges Verfahren bereitgestellt werden als das bisher meistens eingesetzte radiologische Sentinel-Lymphknoten-Diagnostizierverfahren. Ferner soll erfindungsgemäß eine Zusammensetzung bereitgestellt werden, die sich für ein solches Verfahren einsetzen lässt.

Dieses Ziel wird erfindungsgemäß erreicht durch Bereitstellung eines nicht-radiomarkierten Kontrastmittels zur Lymphknoten-Darstellung, das als wesentlichen Bestandteil physiologisch verträgliche und pharmazeutisch unbedenkliche Partikel enthält, die zu 90% (bezogen auf die Teilchenzahl) oder mehr eine Teilchengröße im Bereich von 5 bis 1500 nm aufweisen und eine Farbe haben, die sich mit bloßem Auge von der Farbe menschlichen Gewebes unterscheiden lässt.

Vorzugsweise sind die Teilchen mit einem pharmazeutisch akzeptablen Farbstoff angefärbt, der entweder im Bereich des sichtbaren Lichts oder im UV wenigstens ein Wellenlängenabsorptions- oder Emissionsmaximum hat, das bei entsprechender Beleuchtung seine Unterscheidung von menschlichem Gewebe mit bloßem Auge erlaubt. Besonders bevorzugt haben die angefärbten Partikel bzw. der Farbstoff, mit dem sie angefärbt wurden, ein Absorptionsmaximum im Bereich von 550 bis 680 nm oder ein Emissionsmaximum im Bereich von 380 bis 780 nm nach Anregung im UV/VIS-Bereich.

Ferner wird erfindungsgemäß ein Verfahren zur Herstellung eines solchen Kontrastmittels bereitgestellt. Nach einem weiteren Aspekt der Erfindung wird ein Verfahren zur Entfernung von Sentinel-Lymphknoten zur Verfügung gestellt, bei dem man einem Patienten vor der Tumoroperation das erwähnte Kontrastmittel in die unmittelbare Umgebung des Tumors injiziert und während der Operation den angefärbten Sentinel-Lymphknoten diagnostiziert und herausoperiert.

### Partikel

Die erfindungsgemäßen Partikel weisen zu 90 % oder mehr, vorzugsweise zu über 95 % eine Teilchengröße im Bereich von 5 bis 1500 nm, vorzugsweise 25 bis 1000 nm, stärker bevorzugt 30 bis 600 nm und besonders bevorzugt 50-100 nm auf. Andere denkbare und für die Erfindung geeignete Größenbereiche ergeben sich aus beliebigen Kombinationen von Ober- und Untergrenzen dieser (allgemeinen oder bevorzugten) Bereiche, z.B. 30-1500 nm, 25-100 nm oder 100-600 nm. Partikel in diesem Größenbereich werden zwar durch die Lymphkanäle transportiert, bleiben aber in den Lymphknoten hängen.

Die Partikel für das erfindungsgemäße Kontrastmittel können (näherungsweise) globuläre oder nicht globuläre Partikel sein. Es kann sich bei den Partikeln um Proteinpartikel oder Nicht-Proteinpartikel handeln.

Für Nicht-Proteinpartikel ist die Einstellung und Bestimmung der Partikelgröße durch fraktionierte Filtration durch Membranfilter zu empfehlen. Bei Verwendung von Filtern des MF-Typs der Firma Millipore kommen z.B. solche mit den Porengrößen 25, 50, 100, 220, 300, 450, 650, 800 und 1.200 nm in Frage.

Bei gefärbten Proteinpartikeln können sich durch Adsorption auf den Membranen Beläge bilden, die dazu führen, dass die Partikel nicht mehr ihrer Größe entsprechend fraktioniert werden. Hier ist es günstiger, die Filtrationsmethode nur zur Abtrennung von freiem ungebundenem Farbstoff oder von sehr kleinen Partikeln einzusetzen, z.B. durch Verwendung von Filtern mit 25 nm Porenweite. Für die Ermittlung der Partikelgrößenverteilung ist hier die lichtmikroskopische Methode besser geeignet. Dazu wird ein Tropfen der Suspension nativ auf einem Objektträger verbracht, mit einem Deckglas abgedeckt und im Phasenkontrastverfahren bei 1.000facher Vergrößerung beurteilt (z.B. mit Objektiv 100x und Okular 10x unter Verwendung von Immersionsöl). Zur Ermittlung der Partikelgröße mittels Skalierung ist eine entsprechende Software einzusetzen (z.B. Analysis@ der Software Imgaging System GmbH). Es sollten mindestens 500 Teilchen vermessen werden. Werden Fluoreszenzfarbstoffe gebunden, ist der Einsatz der Fluoreszenzmikroskopie unter gleichen Bedingungen von Vorteil. Dadurch werden nur die farbstoffhaltigen Partikel sichtbar, so dass farbstofffreie Teilchen und eventuell vorhandene Verunreinigungen nicht irrtümlich mit erfasst werden können. Die mikroskopische Methode erlaubt darüber hinaus eine Beurteilung der Partikelform (bei sphärischen Partikeln entspricht die Teilchengröße dem geometrischen Durchmesser; bei nicht-sphärischen Partikeln entspricht die Teilchengröße der Mittelung aus der längsten Seite L und der kürzesten Seite K, d.h. (L+K)/2). Außerdem erlaubt die Methode, unerwünschte Artefakte wie z.B. Agglomerate zu erkennen. Eventuell vorhandene Agglomerate werden bei der Teilchengrößenbestimmung nicht berücksichtigt.

Die Partikel für das erfindungsgemäße Kontrastmittel müssen ferner für den Patienten physiologisch verträglich und pharmazeutisch unbedenklich sein, weil sie in vivo verabreicht werden. Die Kriterien für physiologische Verträglichkeit und pharmazeutische Unbedenklichkeit ergeben sich aus den Anforderungen der Arzneimittelbehörden; für die Zwecke dieser Erfindung kann davon ausgegangen werden, dass ein Kontrastmittel bzw. die darin enthaltenen Bestandteile dann physiologisch verträglich und pharmazeutisch unbedenklich ist bzw. sind, wenn das Kontrastmittel von der EMEA oder einer nationalen Gesundheitsbehörde (z.B. FDA in den USA, oder BfArM in Deutschland) für die in vivo Diagnostik von Lymphknoten zugelassen wurde. Die physiologische Verträglichkeit und pharmazeutische Unbedenklichkeit schließt ein, dass das Kontrastmittel in steriler, pyrogenfreier und virenfreier Form vorliegt. Ferner sollten die Partikel nicht immunogen sein und keine toxischen Metaboliten bilden.

Beispiele für Partikel, die solchen Anforderungen genügen, sind dem Fachmann bekannt und werden teilweise bereits in anderen in vivo-Applikationen eingesetzt. Besonders geeignet für solche Partikel sind Polymerpartikel, unter anderem Proteine und polymere Kohlehydrate, z.B. Dextrane, Stärken, unsubstituierte oder substituierte Zellulosederivate, z.B. Acacia, Chitosane, Pectine, Carrageenane, oder Alginate, soweit biokompatibel und in den gewünschten Partikelgrößen erhältlich. Ebenfalls verwendbar sind Partikel auf Basis von Gelatine oder auf Basis von künstlichen Polymeren, z.B. Polyestern, Polyethern, Polyanhydriden, Polyalkylcyanoacrylaten, Polyacrylamiden, Poly(orthoester), Polyphosphazenen, Polyaminosäuren und biologisch abbaubaren Polyurethanen. Insbesondere verwendbar sind Partikel auf Basis von Polylactaten, Polyglycolaten, Poly(epsiloncaprolacton)-polyethern, Polycaprolactonen und ähnlichen Polymeren sowie Copolymeren mit diesen Grundbausteinen. All diese Polymer- und Copolymerpartikel können ggf. oberflächenmodifiziert sein. Der Begriff Proteine schließt hier auch Glycoproteine und Lipoproteine ein.

Weitere Beispiele für prinzipiell verwendbare Partikel sind Poly(alkyl)-methacrylatpartikel, insbesondere Polymethylmethacrylatpartikel, die ggf. noch freie OH- oder Carboxylatgruppen enthalten können, wie offenbart in EP-A-0 240 424, Partikelmischpolymere oder Homopolymere aus Acrylsäure, Acrylsäureestern oder Methacrylsäureestern wie offenbart in WO 96/24377, biokompatible Nanopartikel, deren Oberfläche mit wenigstens einem oberflächenmodifizierenden Mittel behandelt wurde (allerdings ohne Wirkstoff) gemäß WO 96/20698.

Besonders geeignet für den Einsatz in Kontrastmitteln gemäß der vorliegenden Erfindung sind Proteinpartikel und insbesondere Albuminpartikel. Diese können in der gewünschten Teilchengrößenverteilung durch Denaturierung von Albumin, vorzugsweise humanem Serumalbumin, und anschließendem Einstellen der Teilchengrößenverteilung durch z.B. Ultraschallbehandlung hergestellt werden. Die Denaturierung erfolgt vorzugsweise durch Erhitzen, vorzugsweise auf 75 °C bis 100 °C. Anschließend wird die erhaltene Mischung in der Regel auf Raumtemperatur (25 °C) abgekühlt, bevor die Teilchengröße durch z.B. Ultraschallbehandlung eingestellt wird. Um ein Agglomerieren der Albuminpartikel zu verhindern, wird der Zusammensetzung vorzugsweise ein nicht-ionisches Tensid wie Pluronic (z.B. Pluronic F-68) hinzugesetzt. In der erfindungsgemäßen Zusammensetzung liegen vorzugsweise weniger als 10%, besonders bevorzugt weniger als 2%, der insgesamt vorhandenen Partikel in Agglomeraten vor. Entsprechende Verfahren sind in US 4,024,233 und US 4,187,285 beschrieben. Albuminpartikel-Zusammensetzungen mit geeigneter Teichengröße sind auch im Handel erhältlich, z.B. Solco® Nanocoll oder Albu-Res (Hersteller: Amersham Sorin S.r.l., Vercelli, Italien).

Ebenfalls geeignet sind Dextranpartikel entsprechender Größe. Das primäre Produkt wird durch den Lactobazillenstamm *Leuconostoc mesenteroides B512* synthetisiert und deshalb biologisch aus dessen Kulturmedium gewonnen. Es besteht aus α-(1→6)-verknüpften D-Glucopyranosemolekülen, die über α-(1→3)-Bindungen verzweigt sind (J.W. van Cleve, W.C.
Schäfer, C.E. Rist, The structure of NRRLB-512 dextran. Methylation studies, J. Amer. Chem. Soc. 78 (1956) 4435-4438 und S. Lindberg, S. Svenson, Structural studies on dextran from *Leuconostoc mesenteroides* NRRL B-512. Acta Chem. Scand. 22 (1968) 1907-1912). Das aus dem Kulturmedium isolierte Dextran ist ein Gemisch aus sehr verschiedenen Molekulargewichten. Durch partielle Hydrolyse können sehr große Ketten verkürzt und über mehrfache Fraktionierung mittels Ethanol/Wasser-Gemischen die gesuchten Partikelgrößen isoliert werden (B. Ingelman, M.S. Halling, Some physicochemical experiments on fractions of dextran. Ark. Kemi 1 (1949) 61-80).

Besonders geeignet sind Dextranfraktionen mit Molekulargewichten von 2-5 Millionen. Verfügbar ist Dextran 2000 100G (Hersteller: Amersham Biosciences). Das mittlere Molekulargewicht dieses Produkts beträgt ca. 2 Millionen. Es hat eine fadenförmige bis gestreckt-knäuelige Struktur und einen Stokes-Durchmesser von etwa 54 nm (Amersham Biosciences, User Report of DEXTRAN 2000 100G).

Ebenfalls geeignet sind Dextrane höherer Molekulargewichte. Verfügbar ist eine Dextranmischung von 5-40 Millionen (Hersteller: ICN Biomedicals).

Die erfindungsgemäß verwendeten Partikel sind nicht radiomarkiert, d.h. sie enthalten kein radioaktives Element wie z.B. ^{99m}TC.

### Farbstoff

Der Farbstoff zur Verwendung im erfindungsgemäßen Kontrastmittel sollte pharmazeutisch akzeptabel und als Stoff für die in vivo Verabreichung am Menschen zugelassen sein. Seine Funktion ist die der farblichen Markierung des Sentinel-Lymphknotens für den operierenden Arzt, ohne dass dieser zur Erkennung des Sentinel-Lymphknotens auf aufwändige Technologien, z.B. Gammasonden, zurückgreifen muss. Vielmehr sollte der Operateur den bzw. die Sentinel-Lymphknoten mit bloßem Auge ohne weiteres erkennen können. Entsprechend sollte der Farbstoff entweder im Bereich des sichtbaren Lichts oder im UV wenigstens ein Wellenlängenabsorptionsoder Emissionsmaximum haben, das bei entsprechender Beleuchtung (entweder mit normalem Licht oder UV-Licht) seine Unterscheidung von menschlichem Gewebe mit bloßem Auge erlaubt. Vorzugsweise hat der Farbstoff ein Absorptionsmaximum im Bereich von 550 bis 680 nm und/oder ein Emissionsmaximum im Bereich von 380 bis 780 nm nach Anregung im UV/VIS-Bereich. Besonders bevorzugt sind dunkelblaue oder intensiv grüne Farbstoffe. Fluoreszenzfarbstoffe kommen aber ebenfalls in Frage.

Farbstoffe aus zahlreichen Farbstoffklassen eignen sich zum Einsatz in den erfindungsgemäßen Kontrastmitteln. Besonders bevorzugt sind Azofarbstoffe, Bisazofarbstoffe, Trisazofarbstoffe, Diarylmethan-, Triarylmethanfarbstoffe, Anthrachinonfarbstoffe, polyzyklische aromatische Carbonylfarbstoffe, Indigofarbstoffe, Phthalocyanine, Nitrosofarbstoffe und Triphenodioxazinfarbstoffe.

Ganz besonders bevorzugt sind konkret z.B. die Farbstoffe Acid blue 40, Acid green 27, Amido black, Chicago sky blue, Cibacron blue F3G-A, FR, FN-R, P-3R, Cibacron brillant blue H-GR, Evans-Blue, Fluoresceine, Indigocarmin, Indigotetrasulfonat, Isosulfan blue, Methylenblau, Monastral blue, Patentblau V, Rhodamine und Trypanblau.

Die Anfärbung der erfindungsgemäßen Partikel durch den Farbstoff kann durch chemische und/oder physikalische Wechselwirkungen (Chemisorption oder Physisorption) vermittelt werden. Unter dem Gesichtspunkt der Stabilität sind kovalente chemische Bindungen zwischen Partikel und Farbstoff bevorzugt. Aber auch physikalische Wechselwirkungen, z.B. Protein-Liganden-Wechselwirkungen, hydrophobe Wechselwirkungen, Wasserstoffbrücken-Bildung u.ä. können für eine zufriedenstellende Anfärbung der Partikel völlig ausreichen, solange der Farbstoff-Partikel-Komplex auch in vivo hinreichend stabil bleibt. Ein praktisches Maß für im Sinne der Erfindung ausreichende Stabilität bzw. Bindungsfestigkeit von Farbstoff an Partikel ist beispielsweise, dass in der für die Applikation vorgesehenen Flüssigkeit in 24 Stunden bei 37°C nicht mehr als maximal 5 Gew.-% des ursprünglich gebundenen Farbstoffs von den Partikeln abgelöst (freigesetzt) werden.

Im Sinne der Erfindung sind Partikel, die durch einen Farbstoff angefärbt sind, auch Partikel, die im Molekül selbst Chromophore enthalten und daher eine Eigenfarbe aufweisen. In diesem Fall kann eine weitere Anfärbung durch einen extern zugegebenen Farbstoff natürlich unterbleiben.

Für die Zwecke dieser Erfindung geeignete Bisazofarbstoffe sind substituierte Aromaten mit zwei Azogruppen, die im sichtbaren Bereich ein Absorptionsmaximum im Bereich von Wellenlängen zwischen etwa 550 und 680 nm haben. Farbstoffe mit einer solchen Absorptionswellenlänge sind in der Regel blau oder grün und somit für die Anwendung bei der Lymphknotendarstellung besonders kontrastreich. Die Extinktion des Farbstoffs bei der maximalen Absorptionswellenlänge sollte vorzugsweise so hoch sein, dass man den Farbstoff mit bloßem Auge im Gewebe gut erkennen kann, wenn er in der zur Injektion üblichen Menge appliziert wird.

Bisazofarbstoffe eignen sich in besonderem Maße für die Anfärbung von Partikeln, insbesondere wenn die Partikel Proteine sind (siehe B.Stopa et al., Supramolecular ligands: Monomer structure and protein ligation capability; Biochimie (1998) 80, 963-968). Viele Bisazofarbstoffe weisen nämlich eine hohe Bindefähigkeit an Protein auf. Besonders bevorzugt ist ein molares Farbstoff-Protein-Verhältnis von mindestens 20, besonders bevorzugt 20 bis 90. Mit humanem Immunglobulin G als Standardprotein wird dieser Wert wie folgt bestimmt (siehe B. Stopa et al., Biochimie (1998) 80, 963-968): Eine Mischung aus Protein (Human-IgG) und Farbstoff (100-facher molarer Überschuss) werden 20 Minuten lang bei 63°C für die Agglutination erhitzt (Rollett A., Bacher W., Azo dyes IV, Monatsh. 73 (1940), 20-24). Das Farbstoff-Protein-Verhältnis wird dann spektrophotometrisch in Proben gemessen, die durch eine Bio-Gel P-6-Säule in 0,1 M Phosphatpuffer (pH 7,4; NaCl 0,15 M) zur Entfernung von überschüssigem Farbstoff filtriert wurden. Der Messung geht ein Trypsin-Verdauungsschritt zur Lösung des Farbstoffes vom Protein voraus.

Evans blue(4,4'- Bis(1-amino-8-hydroxy-2,4-disulfo-7-naphthylazo)-3,3'-bitolyl-Tetranatriumsalz) ist aufgrund seiner starken Bindung an Albumin und andere Proteine sowie aufgrund seiner physiologischen Verwendbarkeit (siehe z.B. El-Sayed et al., Re-evaluation of Evans blue dye dilution method of plasma volume measurement, Clin.Lab.Haem. 1995, 17, 189-194) in besonderer Weise als Farbstoff in den erfindungsgemäßen Kontrastmitteln geeignet. Evans blue wurde bereits in ungebundener Form für die Lymphknotendarstellung verwendet (siehe J.-Y. Bobin et al., Tagging Sentinel Lymph Nodes: a Study of 100 Patients with Breast Cancer, Eur.J.Cancer, Ausgabe.35, Nr.4, 569-573, 1999). Die Verwendung von Evans blue für die Lymphknotendarstellung wurde jedoch später als nachteilig beschrieben, insbesondere gegenüber Patentblau, und daher hat sich in der Praxis letzteres durchgesetzt (siehe z.B. Bachter et al., Die Sentinel-Lymphonodektomie beim malignen Melanom, Der Nuklearmediziner, 22, V245-252 1999). Bisher wurden sowohl Patentblau als auch Evans Blue allerdings immer in ungebundener Form direkt verwendet, während ihre Verwendung zum Anfärben von Partikeln und der Einsatz solcher Partikel für die Lymphknoten-Darstellung nicht beschrieben oder vorgeschlagen wurde.

### Herstellung von angefärbten Protein-Partikeln

In einer besonders bevorzugten Ausführungsform bestehen die Partikel aus einem denaturierten Protein, insbesondere humanem Serum-Albumin, und der Farbstoff ist,ein AzoFarbstoff, insbesondere ein Farbstoff mit zwei Azogruppen (Bisazofarbstoff). Die Anfärbung des Proteins durch den Azofarbstoff kann vor, während oder nach der Denaturierung des Proteins erfolgen. Letzteres trifft im Falle von humanem Albumin z.B. bei Verwendung handelsüblicher Partikel zu. Hierzu werden Partikel in z.B. Phosphatpuffer (8,2 mM Na₂HPO₄, 1,8 mM KH₂PO₄, 137 mM NaCl, pH 7,2) in einer Konzentration entsprechend 0,4 bis 4 mg (vorzugsweise z.B. etwa 2,5 mg bei Albu-Res bzw. etwa 0,5 mg bei Solco Nanocoll) Albumin pro ml Lösung suspendiert.

Im allgemeinen kann man einen Farbstoff an die erfindungsgemäßen Partikel binden, indem man zunächst eine Suspension mit der gewünschten Partikelgröße herstellt und dieser dann den Farbstoff in Lösung zufügt. Bevorzugt sind Farbstoffkonzentrationen von 1 bis 10 mM, vorzugsweise 3 bis 5 mM (z.B. 4 mM). Die Mischung wird vorzugsweise mindestens 15 min bei Raumtemperatur stehen gelassen. Mit Farbstoff markierte (angefärbte) Proteinpartikel werden dann von überschüssigem freien Farbstoff abgetrennt, z.B. durch:
a) Säulenchromatographie, z.B. über Sephadex G-50 fine (Pharmacia) mit Phosphatpuffer. Der separierte Partikel/Farbstoff-Komplex kann bei Bedarf schonend eingeengt werden, z.B. mittels Vakuum-Zentrifugalverdampfer.
b) Filtration durch Filter mit einer Porengröße, die deutlich unterhalb der Partikelgröße aber oberhalb der des freien Farbstoffs liegt. Bei Verwendung von Filterkammern (z.B. von Millipore) wird Phosphatpuffer der o.g. Zusammensetzung nachgesetzt. Die Prozedur ist auch geeignet, den Phosphatpuffer bei Bedarf durch beliebige andere Lösungen zu ersetzen.

Ganz analoge Verfahren können auch für viele andere Protein-Farbstoff-Kombinationen verwendet werden.

Eine weitere Möglichkeit zur Herstellung von angefärbten Partikeln ist eine chemische Reaktion der Partikel mit einem geeigneten Reaktivfarbstoff in einem geeigneten Lösungsmittel.

Geht man von einem nativen Protein, insbesondere Albumin, aus, wird dieses zunächst vorzugsweise in z.B. Phosphatpuffer gelöst. Die Lösung wird dann zur Denaturierung des Proteins auf eine geeignete Temperatur erhitzt, z.B. auf 75 bis 100°C im Fall von Albumin. Nach Abkühlung der so erhaltenen Suspension wird der Farbstoff hinzugegeben. Die Mischung kann dann gerührt werden. Das gefärbte denaturierte Protein wird dann abgetrennt, z.B. durch Zentrifugation. Nach Aufnahme des Proteins in Wasser wird die so erhaltene Suspension dann einer Ultraschallbehandlung unterworfen, um die Partikelgrößenverteilung einzustellen. Eine geeignete Dauer und Stärke der Behandlung kann durch Bestimmung der Ausbeute an Partikeln geeigneter Größe erfolgen. Dazu wird die weiter vorn beschriebene lichtmikroskopische Methode empfohlen.

In einem ganz besonders bevorzugten Aspekt betrifft die vorliegende Erfindung ein Kontrastmittel für die Markierung von Sentinel-Lymphknoten im menschlichen Gewebe, enthaltend Albuminpartikel, die zu über 95% eine Größe im Bereich von 25-1000 nm haben und mit dem Farbstoff Evans Blue angefärbt sind, in steriler und pyrogenfreier Form in einem pharmazeutisch akzeptablen wässrigen Lösungsmittel. In bestimmten Ausführungsformen kann dieses Kontrastmittel zusätzlich auch Albuminpartikel enthalten, die mit radioaktivem 99m Tc markiert sind (siehe unten).

### Herstellung anderer Partikel-Farbstoff-Kombinationen

Allgemeine Herstellungsbeispiele für geeignete Partikel aus synthetischen Polymeren gemäß der Erfindung lassen sich z.B. aus den Ausführungsbeispielen der schweizerischen Patentanmeldung CH-614 856 ableiten, wobei lediglich im letzten Schritt das "biologisch oder pharmakodynamisch aktive Material" durch einen geeigneten Farbstoff im Sinne obiger Definition ersetzt wird oder der Farbstoff mit in die Polymerisation einbezogen wird.

### Herstellung des endgültigen Kontrastmittels

Nach der Herstellung von geeigneten angefärbten Partikeln können diese mit weiteren Formulierungshilfsmitteln (Additiven) versetzt und anschließend für die Lagerung konserviert werden, z.B. durch Sterilisierung und gegebenenfalls Lyophilisierung, so dass man - ggf. nach Auflösung in physiologischer Kochsalzlösung oder einem anderen geeigneten Lösungsmittel - ein anwendungsfertiges Kontrastmittel erhält.

Beispiele für solche Verfahren sind z.B. in US 4,187,285 und US 4,024,233 für Albuminpartikel beschrieben. Die Zusammensetzungen werden vorzugsweise lyophilisiert und unter Stickstoffatmosphäre verpackt.

Die Erfindung stellt daher auch ein anwendungsfertiges Kontrastmittel zur Detektion von Sentinel-Lymphknoten bereit, das die erfindungsgemäßen angefärbten Partikel in steriler und pyrogenfreier Form enthält. Die Formulierung sollte aus medizinischen und zulassungsrechtlichen Gründen insbesondere frei von Hepatitis B-Oberflächenantigenen (HbsAg), Antikörpern für das humane Immundefizienz-Virus (anti-HIV 1/2) sowie Antikörpern für den Hepatitis C-Virus (anti-HCV) sein. Die Formulierung kann in Form einer anwendungsfertigen physiologisch akzeptablen Lösung, die vorzugsweise isotonisch sein sollte, bereitgestellt werden, oder in lyophilisierter Form.

Beispielsweise kann eine geeignete Formulierung Glucose, ein nichtionisches Tensid, das die Agglomeration verhindert, wie z.B. Pluronic (z.B. Pluronic F-68), Phosphatpuffer (Natriumphosphat) und/oder Natriumphytat enthalten. In der einfachsten Variante enthalten die erfindungsgemäßen Kontrastmittel jedoch lediglich die erfindungsgemäß angefärbten Partikel in einem geeigneten isotonischen Puffersystem, z.B. Phosphatpuffer pH 7.4 oder Trispuffer pH 8.0, oder in isotonischer Kochsalzlösung.

### Anwendung der Kontrastmittel in der Tumorbehandlung

Die Erfindung betrifft auch die Anwendung der erfindungsgemäßen Kontrastmittel für die Lymphknotendarstellung, insbesondere die Darstellung des bzw. der Sentinel-Lymphknoten. Die erfindungsgemäßen Kontrastmittel kommen vorzugsweise zum Einsatz während der operativen Entfernung eines Tumors, insbesondere eines Melanoms, Mammakarzinoms, Zervixkarzinoms oder Prostatakarzinoms.

Die Verwendung des erfindungsgemäßen Kontrastmittels ist insbesondere dann vorteilhaft, wenn die Lymphabflussrichtung des Tumors bekannt ist. Das ist - abhängig von der Art und der Lokalisierung des Tumors - oft der Fall. Die erfindungsgemäßen Kontrastmittel können unmittelbar vor der Operation (abhängig von Art und Sitz des Tumors in der Regel zwischen 10 min und 2 h vorher) injiziert werden, so dass der bzw. die Sentinel-Lymphknoten während der Operation durch ihre Anfärbung mit den erfindungsgemäßen Partikeln mit bloßem Auge erkannt werden können. Der gesamte technische, finanzielle und personelle Aufwand der Radionuklidtechnik kann dann entfallen.

Bei dieser erfindungsgemäßen Verwendung wird einem Patienten vor der Operation eine erfindungsgemäße pharmazeutische Zusammensetzung mit angefärbten Partikeln als Kontrastmittel durch Injektion in den Tumor oder in seine unmittelbare Umgebung verabreicht. Dann wird eine ausreichende Zeit zugewartet, damit die angefärbten Partikel den Sentinel-Lymphknoten sicher erreichen. Abhängig von Art und Sitz des Tumors liegt dies zwischen 10 min und 2,h. Während der Operation wird dann der Tumor zusammen mit dem oder den angefärbten Sentinel-Lymphknoten entfernt. Je nach dem Zustand der Sentinel-Lymphknoten kommt dann die operative Entfernung weiterer Lymphknoten in Betracht. Wenn der Sentinel-Lymphknoten metastasenfrei ist, ist es nicht erforderlich, noch weitere Lymphknoten zu entfernen.

Die Erfindung wird durch die nachstehenden nichtbeschränkenden Beispiele erläutert.

### Beispiele

### Beispiel 1:

Herstellung einer erfindungsgemäßen Albuminpartikel-Zusammensetzung - ausgehend von Albu-Res (Nycomed Amersham Sorin)
1. 2,5 mg Albumin als Mikrokolloid aus einer Flasche Albu-Res werden in 1 ml Phosphatpuffer (8,2 mM Na₂HPO₄, 1,8 mM KH₂PO₄, 137 mM NaCl, pH 7,2) suspendiert.
2. 0,5 ml dieser Suspension werden mit 2 mg Evans blue (Merck) versetzt, gemischt und 15 min bei Raumtemperatur stehen gelassen.
3. Danach wird die Suspension auf eine Säule (d = 1 cm, h = 2,5 cm) aufgetragen, die mit Sephadex G-50 fine (Pharmacia) gefüllt ist, das mit dem Phosphatpuffer der o.g. Zusammensetzung äquilibriert ist.
4. Elution mit dem gleichen Phosphatpuffer und Fraktionierung zu je 1 ml. Der Evans blue/Albu-Res-Komplex erscheint im Außenvolumen.
5. Die Gipfelfraktionen (Nr. 2 bis 5),werden vereint und mittels Vakuum-Zentrifugalverdampfer auf 0,4 bis 0,5 ml konzentriert.

### Beispiel 2:

Herstellung einer erfindungsgemäßen Albuminpartikel-Zusammensetzung - ausgehend von humanem Serumalbumin
1. 2 ml eines 0,5 M Phosphatpuffers vom pH 5,4 werden mit bidestilliertem Wasser auf 100 ml aufgefüllt und 0,4 g humanes Serumalbumin zugesetzt (z.B. 2 ml Humanalbumin Kabi 20 %ig).
2. Die Lösung wird im Wasserbad unter ständigem Rühren für 20 min auf 80°C erhitzt und danach auf Raumtemperatur zurückgekühlt.
3. Zugabe von 0,2 g Evans blue (Merck) und 15 min bei Raumtemperatur unter leichtem Rühren stehenlassen.
4. Zentrifugation der Mischung und Verwerfen des Überstandes. Auffüllen mit bidestilliertem Wasser auf etwa das ursprüngliche Volumen und Resuspendieren des Niederschlages. Dann erneute Zentrifugation und Wiederholung dieser Prozedur bis im Überstand keine nennenswerte Blaufärbung mehr auftritt.
5. Aufnahme der präzipitierten Aggregate in ca. 100 ml bidestilliertem Wasser und Behandlung mit Ultraschall. Dauer und Stärke der Ultraschallbehandlung sind von vielen Faktoren abhängig und daher zu erproben. Kriterium ist die Ausbeute an Partikeln geeigneter Größe (s. Punkt 7). Typischerweise entstehen überwiegend sphärische Partikel von etwa 450 nm Durchmesser, mit einer Schwankungsbreite von 300 bis 600 nm.
6. Zur Abtrennung von Resten freien Farbstoffs'und eventuell vorhandener zu kleiner Partikel erfolgt eine Filtration der Partikelsuspension durch Filter mit einer Porengröße von 25 nm unter Nachfüllen/mit physiologischer Kochsalzlösung, so daß ein Endvolumen von ca. 25 ml eingestellt wird.
7. Ermittlung der Partikelgrößenverteilung im Rückstand über dem Filter durch Untersuchung einer Probe im Phasenkontrast- und/oder Fluoreszenzmikroskop mit Skalierung bei 1.000facher Vergrößerung, wie auf Seite 6 beschrieben.

### Beispiel 3:

Herstellung einer erfindungsgemäßen Dextranpartikel-Zusammensetzung - ausgehend von Dextran mit einem Molekulargewicht von 5-40 Millionen
1. 1 g Dextran (Molekulargewicht 5-40 Millionen, Hersteller: ICN Biomedicals) werden mit 20 ml bidestilliertem Wasser versetzt und unter Rühren bei Raumtemperatur ca. eine Stunde stehen gelassen.
2. Behandlung mit Ultraschall unter Kühlung bis die Suspension klar ist.
3. Zusatz von 100 mg des Trisazofarbstoffes CIBACRON Blau P-3R (Hersteller: Ciba) in 5 ml bidestilliertem Wasser gelöst und 15 min bei Raumtemperatur rühren.
4. Zusatz von 0,5 g NaCl und 30 min rühren.
5. Zusatz von 0,5 ml einer 5N NaOH, rühren und 20 Stunden bei Raumtemperatur stehen lassen.
6. Neutralisieren mit 5N HCl
7. Zentrifugieren (3.000xg), Überstand verwerfen und Niederschlag in 25 ml bidestilliertem Wasser resuspensieren.
8. Wiederholung von Schritt 7. bis der Überstand keine nennenswerte Blaufärbung mehr zeigt und Aufnahme des Niederschlages in 20 ml physiologischer Kochsalzlösung.

### Beispiel 4:

Herstellung einer erfindungsgemäßen Dextranpartikel-Zusammensetzung - ausgehend von Dextran mit einem Molekulargewicht von etwa 2 Millionen
1. 1 g Dextran T-2000 (Molekulargewicht ca. 2 Millionen, Hersteller: Amersham) wird mit 20 ml bidestilliertem Wasser versetzt und unter Rühren bei Raumtemperatur ca. eine Stunde stehen gelassen.
2. Zusatz von 100 mg des Trisazofarbstoffes CIBACRON Brillant Blau H-GR (Hersteller: Ciba) in 5 ml bidestilliertem Wasser gelöst und 15 min bei Raumtemperatur rühren.
3. Zusatz von 0,5 g NaCl und 30 min rühren.
4. Zusatz von 0,5 ml einer 5N NaOH, rühren und 20 Stunden bei Raumtemperatur stehen lassen.
5. Neutralisieren mit 5N HCl
6. Filtration durch ein Membranfilter mit einer Porenweite von 25 nm und Nachwaschen mit bidestilliertem Wasser, bis der Durchlauf frei von Farbstoff ist.
7. Auffüllen des Filtrationsrückstandes mit physiologischer Kochsalzlösung auf 20 ml.

### Beispiel 5:

### Herstellung einer erfindungsgemäßen Polymethylmethacrylatpartikel-Zusammensetzung

1. 50 ml einer physiologischen Kochsalzlösung werden mit 10 mg des im UV-Licht fluoreszierenden Triarylmethanfarbstoffes DY 555 (Hersteller: Dyomics) versetzt und mit Stickstoff begast.
2. Zusatz von 0,4 ml Methacrylsäuremethylester (Hersteller: Roth) und erneute Begasung mit Stickstoff.
3. Zusatz von 30 mg Peroxydisulfat (Hersteller:Roth) und Polymerisation durch UV-Bestrahlung unter Kühlung für eine Stunde.
4. Zentrifugation mit 1000 xg für 10 min. Verwerfen des Überstandes und Aufnahme des Sediments mit 50 ml bidestilliertem Wasser.
5. Wiederholung von Schritt 4.
6. Aufnahme des Sediments in 25 ml bidestilliertem Wasser und Behandlung mit Ultraschall unter Kühlung.
7. Filtration durch ein Membranfilter mit einer Porenweite von 25 nm und Nachwaschen mit bidestilliertem Wasser, bis der Durchlauf weitgehend frei von Farbstoff ist.
8. Auffüllen des Filtrationsrückstandes mit physiologischer Kochsalzlösung auf 20 ml.

### Beispiel 6:

Tierexperimentelle Darstellung von Sentinel-Lymphknoten Dieses Beispiel dient zur experimentellen Validierung der Brauchbarkeit einer gegebenen Partikel-Farbstoff-Kombination durch Vergleich der durch Anfärbung erhaltenen Lymphknoten-Markierung mit der nach einem radiologischen Standardverfahren erhaltenen radioaktiven Markierung. Obwohl in diesem Beispiel speziell die Kombination Albumin (Albures)-Evans Blue eingesetzt wurde, lassen sich völlig analog beliebige weitere Farbstoff-Partikel-Kombinationen experimentell validieren. So zeigen die Dextran/Cibacronblau-Komplexe gemäß den Beispielen 3 und 4 die gleichen Markierungsmuster wie 99m Technetium oder die in diesem Beispiel verwendeten Evans blue-markierten Albures-Partikel.

Versuchstiere sind weibliche Wistar-Ratten (Alter: 3 Monate, Gewicht 220 - 270 g).
1. Die Narkose wird mit Ether eingeleitet. Dann erhält das Tier Ketamin+Rompun (Mischungsverhältnis 1+1; 1 µl/g Körpergewicht) intramuskulär injiziert. Ketamin 100 mg/ml (Atarost); Rompun: 2 % (Bayer Vital)
2. In die rechte Vorder- und Hinterpfote der Tiere werden je 0,1 ml der gemäß Beispiel 1 hergestellten Zusammensetzung, der das zur Sentinel-Lymphknoten-Darstellung in klinischem Einsatz befindliche ^{99m}Technetium-markierte Albu-Res in einer Dosis von 1 MBq beigemischt ist, subkutan in den Ballen injiziert. Gleichzeitig werden in die linke Vorder- und Hinterpfote der Tiere je 0,1 ml der letztgenannten Zusammensetzung, ebenfalls in einer Dosis von 1 MBq, injiziert.
3. Nach einer Verteilungszeit von 60 min wird das Tier mit einer Überdosis an Ether getötet.
4. Die Lymphknoten beider Seiten werden frei präpariert, entfernt und entsprechend der Nummerierung in der Abbildung FIG.J-1 in: Hebel H, Stromberg MW [eds.] Anatomy and Embryology of the Laboratory Rat, BioMed Verlag Wörthsee, 1986, mit 1 bis 19b beziffert und geordnet.
5. Die Blaufärbung der Lymphknoten wird mit dem bloßen Auge durch den Beobachter bewertet (0: keine Färbung; 1: schwache Färbung; 2: starke Färbung) und die aufgenommene Radioaktivitätsmenge in einem Gamma-Counter gemessen.

Das Ergebnis eines repräsentativen Versuches ist in den Tabellen 1 und 2 dokumentiert, bezüglich der Radioaktivität (RA) einmal als Absolutwerte (Tabelle 1) und zum anderen in Prozent der gesamten gemessenen Radioaktivität (Tabelle 2). Daraus ergibt sich, dass sich Radioaktivität und Blaufärbung nach gleichem Muster verteilen und daher mit dem Partikel/Farbstoff-Komplex die Sentinel-Lymphknoten genau so sicher erfasst werden wie mit dem radiologischen Verfahren. Dies trifft ausnahmslos auf alle bisher dazu durchgeführten Tierversuche zu. Auch Partikel/Farbstoff-Komplexe, die nach in den Beispielen 2 bis 5 aufgeführten Verfahren hergestellt wurden, zeigen das gleiche Verteilungsmuster wie der Albu-Res/Evans blue-Komplex.

**Tabelle 1**

| Versuche mit ^{99m}Tc- und Evans-Blue-markiertem Albures rechts: Evansblue/Albures + ^{99m}Tc/Albures; links: nur ^{99m}Tc/Albures | | | |
|---|---|---|---|
| **Tierseite** | **rechts** | | **links** |
| **LK** | **RA (cpm)** | **Färbung** | **RA (cpm)** |
| 1 | 68,2 | 0 | 127,4 |
| 2 | 23,9 | 0 | 31,0 |
| 3 | 8,7 | 0 | 20,5 |
| 4 | - | - | - |
| **5a** | **1.132.749,4** | **2** | **420.716,7** |
| 5b | 97.785,7 | 1 | 108.669,6 |
| 5c | 48.775,6 | 1 | 291.815,6 |
| 6a | 405.990,1 | 2 | 270.372,0 |
| 6b | 74.718,8 | 0 | 1.116,1 |
| 6c | 5.325,6 | 0 | 1.290,7 |
| 7 | 10,0 | 0 | 19,4 |
| 8 | - | - | - |
| 9 | 55,7 | 0 | 58,3 |
| 10 | 38,8 | 0 | 29,1 |
| 11 | - | - | - |
| 12 | 157, 6 | 0 | 48,2 |
| 13* | 335,5 | 0 | 335,5 |
| 14 | 11.185,3 | 0 | 11.967,2 |
| 15 | - | - | - |
| 16* | 3.190,4 | 0 | 3.190,4 |
| 17 | - | - | - |
| 18 | 168.591,6 | 1 | 76.596,4 |
| 19a | 256,7 | 0 | 387,7 |
| 19b | 332,6 | 0 | 363,8 |
| **Summe (cpm)** | **1.949.600,2** | | **1.187.155,6** |
| LK=Lymphknoten; RA=Radioaktivität; cpm=counts per minute LK 13 und 16 liegen medial und sind singulär (RA auf beiden Seiten eingetragen) | | | |

**Tabelle 2**

| Versuche mit ^{99m}Tc- und Evans-Blue-markiertem Albures | | | |
|---|---|---|---|
| rechts: Evansblue/Albures + ^{99m}Tc/Albures; | | | |
| links: nur ^{99m}Tc/Albures | | | |
| **Tierseite** | **rechts** | | **links** |
| **LK** | **RA (%)** | **Färbung** | **RA (%)** |
| 1 | 0,003% | 0 | 0,011% |
| 2 | 0,001% | 0 | 0,003% |
| 3 | 0,000% | 0 | 0,002% |
| 4 | - | - | - |
| **5°** | **58,102%** | **2** | **35,439%** |
| 5b | 5,016% | 1 | 9,154% |
| 5c | 2,502% | 1 | 24,581% |
| 6a | 20,824% | 2 | 22,775% |
| 6b | 3,833% | 0 | 0,094% |
| 6c | 0,273% | 0 | 0,109% |
| 7 | 0,001% | 0 | 0,002% |
| 8 | - | - | - |
| 9 | 0,003% | 0 | 0,005% |
| 10 | 0,002% | 0 | 0,002% |
| 11 | - | - | - |
| 12 | 0,008% | 0 | 0,004% |
| 13* | 0,017% | 0 | 0,028% |
| 14 | 0,574% | 0 | 1,008% |
| 15 | - | - | - |
| 16* | 0,164% | 0 | 0,269% |
| 17 | - | - | - |
| 18 | 8,647% | 1 | 6,452% |
| 19a | 0,013% | 0 | 0,033% |
| 19b | 0,017% | 0 | 0,031% |
| **Summe (cpm)** | **100%** | | **100%** |

## Patentansprüche

1. Nicht-radiomarkiertes Kontrastmittel zur Lymphknoten-Darstellung, enthaltend als wesentlichen Bestandteil physiologisch verträgliche und pharmazeutisch unbedenkliche Partikel, die zu 90% oder mehr eine Teilchengröße im Bereich von 5 bis 1500 nm aufweisen und eine Farbe haben, die sich mit bloßem Auge von der Farbe menschlichen Gewebes unterscheiden lässt.

2. Kontrastmittel nach Anspruch 1, **dadurch gekennzeichnet, dass** die Partikel mit einem pharmazeutisch akzeptablen Farbstoff angefärbt sind.

3. Kontrastmittel nach Anspruch 2, **dadurch gekennzeichnet, dass** der Farbstoff ein Absorptionsmaximum im Bereich von 550 bis 680 nm oder ein Emissionsmaximum im Bereich von 380 bis 780 nm nach Anregung im UV/VIS-Bereich hat.

4. Kontrastmittel nach einem der vorhergehenden Ansprüchen, **dadurch gekennzeichnet, dass** die Partikel zu 90% oder mehr eine Teilchengröße im Bereich von 50 bis 600 nm aufweisen.

5. Kontrastmittel nach einem der vorhergehenden Ansprüche, **dadurch gekennzeichnet, dass** die Partikel aus Proteinen, insbesondere Albumin, Kohlehydraten, insbesondere Dextranen, Stärken, substituierten Zellulosen, Pectinen, Carrageenanen oder Alginaten, oder aus synthetischen Polymeren oder Copolymeren bestehen.

6. Kontrastmittel nach einem der vorhergehenden Ansprüche, **dadurch gekennzeichnet, dass** die Partikel einen Durchmesser von 50 bis 600 nm aufweisen.

7. Kontrastmittel nach einem der vorhergehenden Ansprüche, **dadurch gekennzeichnet, dass** die Anfärbung der Partikel durch die Farbstoffe über hydrophobe, ionische oder kovalente Wechselwirkungen oder über Komplexbildung derart erfolgt, dass die Partikel nicht mehr als maximal 5 Gew.-% des ursprünglich gebundenen Farbstoffes in dem zur Applikation vorgesehenen Lösungsmittel in 24 Stunden bei 37°C freisetzen.

8. Kontrastmittel nach einem der vorhergehenden Ansprüche, **dadurch gekennzeichnet, dass** der Farbstoff ausgewählt ist aus der Gruppe bestehend aus Azofarbstoffen, Bisazofarbstoffen, Triazofarbstoffen, Diarylmethan-, Triarylmethanfarbstoffen, Antrachinonfarbstoffen, Pthalocyaninen, polycyclischen aromatischen Carbonylfarbstoffen, Triphenodioxazinfarbstoffen, Nitrosofarbstoffen und Indigofarbstoffen.

9. Kontrastmittel nach Anspruch 8, **dadurch gekennzeichnet, dass** der Farbstoff ausgewählt ist aus der Gruppe bestehend Acid blue 40, Acid green 27, Amido black, Chicago sky blue, Cibacron blue F3G-A, Cibacron blue FR, Cibacron blue FN-R, Cibacron blue P-3R, Cibacron brillant blue H-GR, Evans-Blue, Fluoresceine, Indigocarmin, Indigotetrasulfonat, Isosulfan blue, Methylenblau, Monastral blue, Patentblau V, Rhodamine und Trypanblau.

10. Kontrastmittel nach einem der vorhergehenden Ansprüche, **dadurch gekennzeichnet, dass** der Farbstoff ein Fluoreszenzfarbstoff ist, der bei Bestrahlung mit UV oder VIS-Licht ein Licht im Bereich von 380-780 nm Wellenlänge emittiert.

11. Kontrastmittel nach einem der vorhergehenden Ansprüche in steriler, pyrogenfreier und lyophilisierter Form.

12. Kontrastmittel nach einem der Ansprüche 1 bis 10 als Partikelsuspension in einem sterilen, pyrogenfreien, pharmazeutisch akzeptablen wässrigen Lösungsmittel.

13. Verwendung eines nicht-radiomarkierten Kontrastmittels nach einem der vorhergehenden Ansprüche zur Darstellung des oder der Sentinel-Lymphknoten eines Tumorpatienten.

14. Verwendung gemäß Anspruch 13, zur Darstellung des oder der Sentinel-Lymphknoten bei einem Melanom, Mamma-, Zervix- oder Prostatakarzinom.

15. Verfahren zur Herstellung einer Zusammensetzung nach einem der Ansprüche 1-12, umfassend die Schritte:
- Umsetzen eines Farbstoffes mit Partikeln, die zu 90 % oder mehr eine Teilchengröße im Bereich von 5 bis 1500 nm aufweisen; und
- Abtrennen des nicht-gebundenen Farbstoffes durch Säulenchromatographie, Zentrifugation oder Filtration.

16. Verfahren zur Herstellung einer Zusammensetzung nach einem der Ansprüche 1 bis 12, wobei die Partikel aus Protein bestehen, umfassend die Schritte:
- Erhitzen einer wässrigen Protein-Lösung auf eine Temperatur, die zur Denaturierung und Agglomerierung des Proteins führt;
- Vermischen des Farbstoffes mit dieser Lösung;
- Abtrennen und Suspendieren des so erhaltenen gefärbten Proteins in Wasser;
- Ultraschallbehandeln der so erhaltenen Suspension zur Herstellung von Partikeln der gewünschten Größe von 5 bis 1500 nm, vorzugsweise 50 bis 600 nm.
- Entfernung eventuell vorhandener zu kleiner Partikel oder von freiem Farbstoff durch Filtration.
- lichtmikroskopische Kontrolle der Partikelgrößenverteilung.

17. Verfahren zur operativen Tumorbehandlung, insbesondere von Mammakarzinomen, Melanomen, Zervix- und Prostatakarzinomen, umfassend die Schritte des Injizierens eines nicht-radiomarkierten Kontrastmittels gemäß einem der Ansprüche 1-12 vor der Operation in den Tumor oder die unmittelbare Tumorumgebung, Zuwarten während eines Zeitraums, der für das Abfließen des Kontrastmittels durch das Lymphsystem bis mindestens zum Sentinel-Lymphknoten ausreicht, und dann operative Entfernung des Tumors und des bzw. der angefärbten Sentinel-Lymphknoten und Überprüfung derselben auf Metastasen.
